# EUROPEAN PATENT SPECIFICATION

(11) **EP 2 915 807 B1**
(45) Date of publication and mention of the grant of the patent: **11.10.2017**
(21) Application number: 13851399.9
(22) Date of filing: 01.11.2013
(51) Int. Cl.: C07D 273/04, C07B 61/00

(54) **METHOD FOR PRODUCING AND STORING LIQUID COMPOSITION COMPRISING TETRAHYDRO-4H-1,3,5-OXADIAZINE-4-ONE**
VERFAHREN ZUR HERSTELLUNG UND LAGERUNG EINER FLÜSSIGEN ZUSAMMENSETZUNG MIT TETRAHYDRO-4H-1,3,5-OXADIAZIN-4-ON
PROCÉDÉ DE PRODUCTION ET DE STOCKAGE D'UNE COMPOSITION LIQUIDE COMPRENANT DE LA TÉTRAHYDRO-4H-1,3,5-OXADIAZINE-4-ONE

(30) Priority: 02.11.2012 JP 2012242897; 02.11.2012 JP 2012242898
(43) Date of publication of application: 09.09.2015
(73) Proprietor: Kao Corporation, Chuo-ku Tokyo 103-8210 (JP)
(72) Inventor: NAGASAWA, Koji, Wakayama-shi Wakayama 640-8580 (JP); KAWAKAMI, Hiroyuki, Wakayama-shi Wakayama 640-8580 (JP); AKINO, Yusuke, Wakayama-shi Wakayama 640-8580 (JP)
(74) Representative: Hoffmann Eitle
(86) International application number: PCT/JP2013/079692
(87) International publication number: WO 2014/069620

(56) References cited:
- EP-A1- 0 013 307
- WO-A1-90/11308
- DE-A1- 2 207 921
- JP-A- H1 160 419
- JP-A- S4 868 583
- JP-A- S5 571 689
- JP-A- H03 163 124
- JP-A- H04 503 824
- OLIVER STEINHOF ET AL: "Quantitative and qualitative 1 H,? 13 C, and 15 N?NMR spectroscopic investigation of the urea-formaldehyde resin synthesis", MAGNETIC RESONANCE IN CHEMISTRY., vol. 52, no. 4, 4 April 2014 (2014-04-04), pages 138-162, XP055265443, GB ISSN: 0749-1581, DOI: 10.1002/mrc.4044
- BUNICHIRO TOMITA ET AL: "Urea-formaldehyde resins: NMR study on base-catalyzed reaction of formaldehyde with urea in deuterium oxide", JOURNAL OF POLYMER SCIENCE, POLYMER CHEMISTRY EDITION., vol. 14, no. 2, 1 February 1976 (1976-02-01), pages 387-401, XP055265568, US ISSN: 0360-6376, DOI: 10.1002/pol.1976.170140211
- BRAUN DIETRICH ET AL: "Die Bildung von Methylolverbindungen bei der Umsetzung von Harnstoff mit konzentrierten Formaldehydlosungen /// Formation of methylol compounds during the reaction of urea with concentrated formaldehyde solutions", ANGEWANDTE MAKROMOLEKULARE CHEMIE. APPLIED MACROMOLECULARCHEMISTRY AND PHYSICS, WILEY VCH, WEINHEIM, DE, vol. 83, 1 January 1979 (1979-01-01), pages 21-36, XP009189509, ISSN: 0003-3146
- TIAN Y ET AL.: 'Study on structure and composition of uron rings compounds under alkalinity condition' ZHONGGUO JIAONIANJI vol. 16, no. 3, 2007, pages 8 - 10, XP008178728

## Description

### Field of the invention

The present invention relates to a method for producing a liquid composition containing tetrahydro-4H-1,3,5-oxadiazin-4-one, as well as a storage solution obtained by the method.

### Background of the invention

Production of various compounds by reacting urea and formaldehyde in water has been performed. Furthermore, a technique for stable storage of urea or formaldehyde as a raw material and a reaction intermediate has been developed.

For example, in JP-A 55-71689, it is disclosed that some kinds of a reaction product between urea and formaldehyde exhibit, as a fertilizer composition, a significant effect for supplying nutrition to leaves of a growing plant. Furthermore, as a condition for production, it is disclosed that an aqueous mixture of urea and formaldehyde in which the molar ratio of urea : formaldehyde is about 1 : 1 or higher and about 2 : 1 or lower is produced, ammonia is added thereto such that it is about 0.3 to 6% of the urea and formaldehyde, the resulting mixture is heated at a temperature between about 75°C and the boiling point, while the pH of the mixture is maintained at high alkali, at about 8.5 to 10, until at least about 90% of the formaldehyde is in a binding form and at least about 60% is in a methylol compound form, and then the heating is continued at pH 7 to 8.5 until at least 50% but about 80% or less of the formaldehyde is in a methylene form. It is also described that, the reaction product is a concentrated aqueous solution containing about 80% to 90% of a partial reaction product of urea and formaldehyde, which is transparent for at least about 30 days at room temperature and does not yield "salting-out", that is, solid-precipitation, for at least 7 days at 0°C.

Further, in the publication of JP-A 4-503824, a urea-formaldehyde condensed product causing, when used as a chemical for wetting paper, less formaldehyde-release during paper drying compared to a condensed product of a related art which is based on urea and formaldehyde is disclosed. It is also disclosed that a) urea and triethanolamine are reacted under ammonia decomposition at a molar ratio of 1 to 6 : 1 and a temperature of 130 to 180°C and b) the reaction product obtained from the step (a) is subjected to methylolation in an aqueous medium in a basic pH range by using formaldehyde.

DE 22 07 921 A1 describes a method for reacting formaldehye and urea in water to produce resins which contain uron-type compounds which principally consist of 4-oxotetrahydro-1,3,5-oxadiazine (uron). Steinhof et al. provide an overview of the reaction of formaldehyde and urea including the formation of uron (Magnetic Resonance in Chemistry, 2014, vol. 52(4), pp. 138-162). Tomita et al. also describe the reaction of formaldehyde and urea to form uron but with different bases (Journal of Polymer Science, Polymer Chemistry Edition, 1976, vol. 14(2), pp. 387-401). EP 0 013 307 A1 describes the reaction between formaldehye and urea in water with ammonia and where additional ammonia is added to the end product before storage. WO 90/11308 A1 describes a reaction where urea and alkanolamine are reacted at a temperature of 130-180°C and are subsequently reacted with formaldehye in basic media in water. Dietrich et al. describe a reaction between formaldehyde and urea in water at temperatures from 30 to 50°C (Angewandte makromolekulare Chemie - Applied macromolecular chemistry and physics, 1979, vol. 83(1), pp. 21-36).

Further, with regard to the production of a melamine-urea resin, JP-A 59-108019 regards a lot of formaldehyde released from the obtained resin as a problem, and discloses a production method wherein 2-dimethyl or 2-diethyl aminoethanol is added to a mixture of 1.0 mol of melamine, 4.0 to 4.5 mol of urea, and 5.5 to 7.5 mol of formaldehyde to alkalify the mixture and to maintain it under reflux at 75°C or higher, and the addition of 2-dimethyl or 2-diethyl aminoethanol maintains the pH of the reaction solution, which does not decrease from 7.5.

In JP-A 48-36223, a hydraulic cement mixture containing additive which contains a water-soluble adduct hydroxylated with a monomer which has been formed by a reaction between aldehyde and urea is described. Among those adducts, an adduct produced as follows is described in the example 1: monomethylol urea in which 1 mol of formaldehyde is condensed with 1 mol of urea is produced by dissolving 50 g of paraformaldehyde in 1 L of water and adding 1 g of triethanolamine and 100 g of urea followed by keeping them for 4 days.

For example, in JP-A 61-136983, as a liquid fertilizer composition, a fertilizer composition containing specific amounts of a triazone composition which is soluble in water, a urea type compound, a methylene diurea-type compound, a monomethylol urea type compound, a hexamethylenetetramine type compound, and water is disclosed. Furthermore, as a novel composition containing a triazone composition which is suitable for an application as a fertilizer, it is disclosed as a composition which exhibits good stability immediately after storage and during storage and also long-term stability against crystallization of the components in the composition or their precipitation from the composition.

In JP-A 3-163124, an aqueous solution of formaldehyde-urea which includes a solution obtained by pre-condensation according to addition of urea to high-concentration aqueous formalin solution and a formalin stabilizer and can be stored at room temperature is disclosed.

Furthermore, in JP-A 55-71689, it is disclosed that some kinds of a reaction product between urea and formaldehyde exhibit, as a fertilizer composition, a significant effect for supplying nutrition to leaves of a growing plant. Furthermore, the reaction product is a concentrated aqueous solution containing about 80% by mass to 90% by mass of a partial reaction product between urea and formaldehyde, which is transparent for at least about 30 days at room temperature and does not yield "salting-out", that is, solid-precipitation, for at least 7 days at 0°C.

### Summary of the invention

The present invention relates to a method for producing, by reacting a compound 1 represented by the following formula (1) and a compound 2 represented by the following formula (2) in water, a liquid composition with a pH of 10.0 or higher containing a compound 3 represented by the following formula (3) and water, and it provides a method for producing a liquid composition containing tetrahydro-4H-1,3,5,-oxadiazin-4-one, in which
the molar feed ratio of the compound 2 to the compound 1, that is, the compound 2/the compound 1, is 0.3 or higher and 1.7 or lower, and
the reaction between the compound 1 and the compound 2 is performed at 15°C or higher and 50°C or lower and a pH of 10.0 or higher in the presence of alkyl diethanolamine.

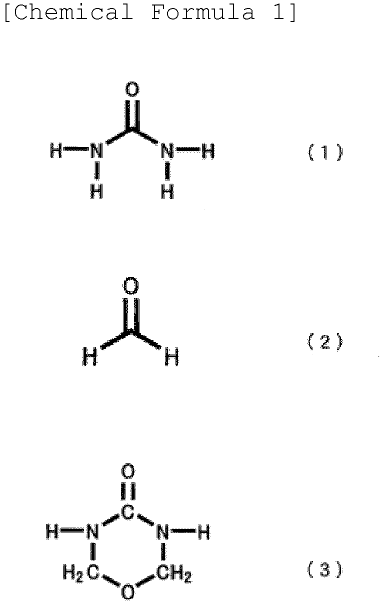

The present invention also provides, as a storage solution of a liquid composition obtained by the aforementioned method for producing a liquid composition described above, a storage solution containing the compound 3, alkyl diethanolamine, and water, in which the content of water is 70% by mass or higher in the storage solution and the pH is 10.0 or higher and 12.5 or lower.

### Detailed description of the invention

According to JP-A 55-71689 and JP-A 4-503824, a reaction between urea and formaldehyde is performed for producing a resin. In a condensed product obtained therefrom, in addition to a monomer in which 1 mol to 2 mol of formaldehyde is condensed per mol of urea, a multimer containing at least 2 mol of urea is produced. However, for using a compound obtained by methylolation of urea with formaldehyde for an application other than a resin, for example, an additive such as a chelating agent, it is preferable that the monomer content is increased as much as possible while reducing the multimer content in order to increase the molecule number per unit mass and increase the chelating power per molecule. From this point of view, for methylolation of urea with formaldehyde, a method for producing a monomer, in which 2 mol of formaldehyde is condensed per mol of urea, at high yield is required. Among them, tetrahydro-4H-1,3,5-oxadiazin-4-one, which is a compound resulting from ring-closure of dimethylol urea in which 2 mol of formaldehyde is condensed per mol of urea, is a compound which is expected to be used as an intermediate of a useful compound such as pharmaceuticals or agrochemicals, or a chelating agent, and thus a method for obtaining it at high yield is required.

Furthermore, for a case where urea is subjected to methylolation with formaldehyde and an aqueous solution of a reactant obtained immediately after the methylolation is stored at room temperature, cloudiness or precipitation of the aqueous solution may occur. Although the reaction product of JP-A 55-71689 has excellent storage stability, it is believed that there is lots of urea contained in the reaction product and the methylol group in the product is capped with urea. A compound having capped methylol group is not suitable for use as a chelating agent. In JP-A 48-36223, there is no description about producing tetrahydro-4H-1,3,5-oxadiazin-4-one at good yield and improving the stability of the aqueous solution.

The present invention provides a method for producing a liquid composition of tetrahydro-4H-1,3,5-oxadiazin-4-one at high yield which enables excellent storage stability of the compound.

According to the present invention, a method for producing a liquid composition of tetrahydro-4H-1,3,5-oxadiazin-4-one [the compound 3 represented by the formula (3)] at high yield enabling excellent storage stability of the compound is provided.

For using a compound obtained by methylolation of urea with formaldehyde for a use other than a resin, for example, an additive such as a chelating agent, it is preferable that the monomer content is increased as much as possible while reducing the multimer content in order to increase the molecule number per unit mass and increase the chelating power per molecule. From this point of view, for methylolation of urea with formaldehyde, a technique for enhancing the stability of a monomer, in which 2 mol of formaldehyde is condensed per mol of urea, is required. Among them, tetrahydro-4H-1,3,5-oxadiazin-4-one, which is a compound resulting from ring-closure of dimethylol urea in which 2 mol of formaldehyde is condensed per mol of urea, is a compound which is expected to be used as an intermediate of a useful compound such as pharmaceuticals or agrochemicals, or a chelating agent, and thus a technique for stable storage of the compound is required.

In JP-A 61-136983, JP-A 3-163124, and JP-A 55-71689, it is described that the reaction product between urea and formaldehyde has excellent storage stability. Meanwhile, it is noted in JP-A 61-136983 that the stability can be enhanced when a triazone composition is contained at high ratio. It is also described that, as monomethylol urea, dimethylol urea, and the like are unstable and have a property of crystallization and precipitation, and their content is rather limited. Furthermore, in JP-A 3-163124 related to providing an aqueous solution as a raw material of an amino resin, it is described that a slight amount of polyvinyl formal is added as a formalin stabilizer and methylol urea capable of being present stably in formalin is pre-condensed under heating. Because methylol urea is pre-condensed, it is considered to be an aqueous solution containing less amount of monomer (methylol urea). According to JP-A 55-71689, there are lots of urea contained in the reaction product and the methylol group in the product is capped with urea and thus in a stabilized state. A compound having methylol group capped with urea is suitable as a raw material for a fertilizer or a urea resin but not suitable for use as a chelating agent. As such, for convenient use of monomethylol urea or dimethylol urea which is useful as a chelating agent, a method for storing them stably in a liquid state is required.

The present invention provides a storage solution of a liquid composition containing tetrahydro-4H-1,3,5-oxadiazin-4-one with excellent storage stability.

Hereinbelow, the method for producing a liquid composition of the present invention is explained in detail.

Although the reason of exhibiting the aforementioned effect by the production method of the present invention remains unclear, it is speculated as follows. For a reaction between urea and formaldehyde in an aqueous solution, it is known that there are two major reactions including methylenation reaction and methylolation reaction. With regard to those reactions, a methylenation reaction having a high molecular weight urea-formaldehyde multimer condensed product as a main component is dominant in an aqueous acid solution. On the other hand, in an aqueous alkali solution, a methylolation reaction having a low molecular weight urea-formaldehyde condensed product containing a monomer as a main component is dominant. In the present invention, by having an alkyl diethanolamine, pH change due to a side reaction such as Cannizzaro reaction of formaldehyde or an occurrence of ammonia caused by alkali decomposition of urea is suppressed and a pH of 10.0 or higher is obtained at 15°C or higher and 50°C or lower. Accordingly, the methyolation reaction can be predominantly progressed. As a result, it is believed that a liquid composition having high content of the compound 3 and low content of a multimer is obtained. It is also believed that, because the multimer has many reaction points to cause a cross-linking reaction with other monomers, the multimer causes a cross-linking reaction during storage to yield high molecular weight and insoluble white precipitates. Furthermore, according to the present invention, because a liquid composition having low content of a multimer and high content of the compound 3, which is a cyclic compound, with low reactivity is obtained and the compound 3 can be stably present as an aqueous solution with a pH of 10 or higher, it is believed that excellent storage stability is obtained.

According to the production method of the present invention, a liquid composition containing the compound 3 represented by the formula (3) and water is obtained by reacting the compound 1 represented by the formula (1) and the compound 2 represented by the formula (2) in water. As a result of the reaction between the compound 1 and the compound 2, a condensed product of the compound 1 and the compound 2 is obtained. One of such condensed products is the compound 3. The compound 1 represented by the formula (1) is urea. The compound 2 represented by the formula (2) is formaldehyde. The compound 3 represented by the formula (3) is tetrahydro-4H-1,3,5-oxadiazin-4-one. The reaction of the compound 1 with the compound 2 is performed by having water as a solvent, and an aqueous solution of reacting materials can also be used.

The molar feed ratio of the compound 2 and the compound 1 according to the production method of the present invention is, in terms of the compound 2/the compound 1, 0.3 or higher and 1.7 or lower. From the viewpoint of enhancing the yield of a condensed product between the compound 2 and the compound 1, it is preferably 0.5 or higher, and more preferably 0.75 or higher and also preferably 1.5 or lower and more preferably 1.25 or lower.

The total feed amount of the compound 1 and the compound 2 is, from the viewpoint of enhancing the yield of a condensed product between the compound 2 and the compound 1, preferably 3.0 parts by mass or higher, more preferably 6.0 parts by mass or higher, and even more preferably 11 parts by mass or higher relative to 100 parts by mass of the total amount of raw materials (the compound 1, the compound 2, alkyl diethanolamine, and water). Furthermore, from the viewpoint of solubility of the compound 3 in water, it is preferably 25 parts by mass or lower, more preferably 20 parts by mass or lower, and even more preferably 14 parts by mass or lower.

The feed amount of the compound 1 is, from the viewpoint of enhancing the yield of a condensed product between the compound 2 and the compound 1, preferably 1.0 part by mass or higher, more preferably 5.0 parts by mass or higher, and even more preferably 8.0 parts by mass or higher relative to 100 parts by mass of the total amount of raw materials (the compound 1, the compound 2, alkyl diethanolamine, and water). Furthermore, from the viewpoint of solubility of the compound 3 in water, it is preferably 20 parts by mass or lower, more preferably 12 parts by mass or lower, and even more preferably 9.0 parts by mass or lower.

The feed amount of the compound 2 is, from the viewpoint of enhancing the yield of a condensed product between the compound 2 and the compound 1, preferably 0.5 part by mass or higher, more preferably 1.0 part by mass or higher, and even more preferably 3.0 parts by mass or higher relative to 100 parts by mass of the total amount of raw materials (the compound 1, the compound 2, alkyl diethanolamine, and water). Furthermore, from the viewpoint of solubility of the compound 3 in water, it is preferably 10 parts by mass or lower, more preferably 8.0 parts by mass or lower, and even more preferably 5.0 parts by mass or lower.

The feed amount of water is, from the viewpoint of solubility of the compound 3 in water, preferably 70 parts by mass or higher, more preferably 75 parts by mass or higher, and even more preferably 80 parts by mass or higher relative to 100 parts by mass of raw materials (the compound 1, the compound 2, alkyl diethanolamine, and water). Furthermore, from the viewpoint of increasing the yield of the compound 3, it is preferably 95 parts by mass or lower, more preferably 90 parts by mass or lower, and even more preferably 88 parts by mass or lower. Meanwhile, for a case of using a raw material other than water in an aqueous solution form, the amount of water added by the aqueous solution to a reaction system is also included in the amount of water as a reaction solvent.

According to the present invention, the reaction between the compound 1 and the compound 2 is performed in the presence of alkyl diethanolamine. Due to the pH buffering activity of alkyl diethanolamine, the pH of the liquid composition can be maintained at 10.0 or higher during the reaction or storage. From the viewpoint of suppressing pH decrease during the reaction, alkyl diethanolamine having an alkyl group with 1 to 3 carbon atoms is preferable among alkyl diethanolamines. Alkyl diethanolamine having an alkyl group with 1 carbon atom, that is, N-methyl diethanolamine, is more preferable. Among alkanolamines, alkanolamine having no alkyl group such as triethanolamine, diethanolamine, or monoethanolamine has low pH increasing activity, low pH buffering activity, and a large decrease in pH during the reaction, and thus the effect of enhancing the yield of the compound 3 or the effect of enhancing the storage stability of an aqueous solution to be obtained is not obtained.

The feed amount of alkyl diethanolamine is, from the viewpoint of enhancing the yield of the compound 3, preferably 0.1 part by mass or higher, more preferably 0.3 part by mass or higher, and even more preferably 0.5 part by mass or higher relative to 100 parts by mass of the total amount of raw materials (the compound 1, the compound 2, alkyl diethanolamine, and water). Furthermore, from the economical viewpoint considering saturated pH buffering activity, it is preferably 5.0 parts by mass or lower, more preferably 1.0 part by mass or lower, and even more preferably 0.6 part by mass or lower.

The molar feed ratio of alkyl diethanolamine to the total amount of the compound 1 and the compound 2 [alkyl diethanolamine/(total of the compound 1 and the compound 2)] is, from the viewpoint of enhancing the yield of the compound 3, preferably 0.005 or higher, more preferably 0.010 or higher, and even more preferably 0.013 or higher. It is also preferably 0.100 or lower, more preferably 0.050 or lower, even more preferably 0.025 or lower, and even more preferably 0.018 or lower.

The reaction temperature for the production method of the present invention is 15°C or higher and 50°C or lower. From the viewpoint of enhancing the reaction rate, it is preferably 20°C or higher, more preferably 35°C or higher, and even more preferably 38°C or higher. Furthermore, from the viewpoint of enhancing the yield of the compound 3, it is preferably 45°C or lower, and more preferably 42°C or lower.

The reaction pH of the production method of the present invention indicates a pH from the start to the end of the reaction. With regard to the reaction between the compound 1 and the compound 2, there is a tendency that the pH decreases in accordance with a progress of the reaction. When a pH controlling agent or the like is not added during the reaction for the production method of the present invention, it is sufficient that the pH at the start of the reaction and the pH at the end of the reaction are within the range of the present invention. When a pH controlling agent or the like is added during the reaction, it is sufficient that, in addition to the pH at the start of the reaction and the pH at the end of the reaction, the pHs before and after adding further a pH controlling agent or the like are within the range of the present invention. The reaction pH in the production method of the present invention is, from the viewpoint of suppressing a cross-linking reaction caused by ring opening of the compound 3 and a decrease in storage stability of the liquid composition, 10.0 or higher. From the viewpoint of enhancing the blending property at the time of using the liquid composition obtained by the present invention, the pH is preferably 13.0 or lower, more preferably 12.0 or lower, and even more preferably 11.5 or lower. Meanwhile, the reaction pH indicates a pH of the reaction system, and it may be a pH of a mixture containing water, the compound 1, the compound 2, and alkyl diethanolamine. Further, with regard to the reaction between the compound 1 and the compound 2 which is the subject of the production method of the present invention, there is a tendency that the pH decreases in accordance with a progress of the reaction. Thus, in the present invention, the pH at the start of the reaction is preferably 10.5 or higher or more preferably 11.0 or higher and preferably 13.0 or lower, more preferably 12.5 or lower, even more preferably 12.0 or lower, and even more preferably 11.5 or lower. The pH after the completion of the reaction is 10 or higher. From the viewpoint of storage stability of the liquid composition, it is preferably 10.5 or higher and more preferably 11.0 or higher. By performing the reaction at a predetermined pH and temperature of the present invention in the presence of alkyl diethanolamine, a liquid composition having a pH of 10.0 or higher can be obtained by the production method of the present invention. If necessary, a pH controlling agent such as acid or base can be added to the liquid composition. In the present invention, it is also possible that the pH is controlled at 10.0 or higher until the end of the reaction by using a pH controlling agent.

The reaction time for the production method of the present invention is, from the viewpoint of enhancing the yield of the condensed product of the compound 2 and the compound 1, preferably 1 hour or longer. More preferably, it is 1.5 hours or longer. Even more preferably, it is 2 hours or longer. From the viewpoint of enhancing the yield of the compound 3, the reaction time is preferably 4.5 hours or shorter, and more preferably 3 hours or shorter.

According to the present invention, it is preferable that the reaction start point is a time point at which the compound 1 and the compound 2 are initially brought into contact with each other and the reaction end point is a time point at which the reduced amount of the compound 2 after 30 minutes is 0.3% by mass or lower relative to the total amount of the raw materials (the compound 1, the compound 2, alkyl diethanolamine, and water). This means that the time point at which the reaction does not substantially progress as no compound 2 is consumed can be taken as the reaction end point. Quantification of the compound 2 in a sample can be obtained by reacting the sample with hydroxylamine hydrochloride followed by titration with sodium hydroxide. Furthermore, in the present invention, although the reaction operation can be immediately terminated at the reaction end point, the reaction operation can be further continued.

Furthermore, from the viewpoint of enhancing the yield of the condensed product of the compound 2 and the compound 1 by preventing a contact of the compound 2 with an alkali solution and suppressing Cannizzaro reaction and also of enhancing the content of the compound 3 by suppressing a rapid reaction to generate a multimer, a method of adding the compound 2 to a mixture containing the compound 1, alkyl diethanolamine, and water, for example, a method in which the compound 1, alkyl diethanolamine, and water are added to a reaction vessel and an aqueous solution containing the compound 2 (formalin) is added dropwise under stirring after adjusting the temperature and pH of the mixture is preferable. It is also preferable that, after the completion of the addition of the compound 2, aging is preferably performed by continuing stirring while the reaction temperature is maintained. When an aqueous solution of the compound 2 is added dropwise, the time required for dropwise addition is preferably 1 hour or longer, and more preferably 1 and half hours or longer from the viewpoint of enhancing the content of the compound 3 based on homogeneous reaction. Furthermore, from the viewpoint of suppressing the aforementioned side reaction, the time required for dropwise addition is preferably 3 hours or shorter, and more preferably 2 hours or shorter. The time for aging is, from the viewpoint of completion of the reaction and homogeneity of the product, preferably 0.5 hour or longer and more preferably 1 hour or longer. Furthermore, from the viewpoint of suppressing the aforementioned side reaction, the time required for aging is preferably 3 hours or shorter, and more preferably 2 hours or shorter. The total time for dropwise addition and aging is, from the viewpoint of enhancing the content of the compound 3, completion of the reaction, and homogeneity of the product, preferably 1.5 hours or longer and more preferably 2 hours or longer. From the viewpoint of suppressing a side reaction, it is preferably 4.5 hours or shorter and more preferably 3 hours or shorter.

In the present invention, a polar solvent (for example, methanol and ethanol) can be used as a reaction solvent in combination in addition to water within a range in which the effect of the present invention is not inhibited. The reaction pressure is not particularly limited. If necessary, the reaction can be performed under increased pressure or reduced pressure. As for the reaction type, any type such as batch type, semi-batch type, and continuous type can be employed. Furthermore, within the range in which the effect of the present invention is not inhibited, an additive such as an anti-foaming agent can be used as a raw material for injection. The ratio of water in the reaction solvent is, from the viewpoint of controlling volatility of a solvent and temperature during the reaction, preferably 50% by mass or higher, more preferably 70% by mass or higher, 90% by mass or higher, or 100% by mass.

The production method of the present invention can also be performed by a method in which an aqueous solution containing the compound 1, the compound 2, and water is prepared in a reaction vessel, a pH of 10.0 or higher is obtained by adjusting an addition amount with addition of alkyl diethanolamine, and the reaction is performed under stirring at a temperature of 15°C or higher and 50°C or lower.

According to the production method of the present invention, content of the compound 3 in the obtained liquid composition is, from the viewpoint of enhancing the storage stability, preferably 1.4% by mass or higher, more preferably 2.0% by mass or higher, even more preferably 2.5% by mass or higher, and even more preferably 3.2% by mass or higher. It is also preferably 30% by mass or lower, more preferably 15% by mass or lower, even more preferably 10% by mass or lower, and even more preferably 8.0% by mass or lower.

According to the production method of the present invention, a liquid composition in which content of the compound 3 represented by the formula (3) is high in a solid matter is obtained. The amount of a solid matter in the liquid composition is, from the viewpoint of enhancing the blending property at the time of using the liquid composition, preferably 5 parts by mass or higher, more preferably 10 parts by mass or higher, and even more preferably 12 parts by mass or higher relative to 100 parts by mass of the liquid composition. From the viewpoint of enhancing the storage stability, it is preferably 30 parts by mass or lower, more preferably 25 parts by mass or lower, and even more preferably 20 parts by mass or lower. The content of water in the liquid composition is, from the viewpoint of enhancing storage stability, preferably 70 parts by mass or higher, more preferably 75 parts by mass or higher, and even more preferably 80 parts by mass or higher relative to 100 parts by mass of the liquid composition. It is also preferably 95 parts by mass or lower, more preferably 90 parts by mass or lower, and even more preferably 88 parts by mass or lower. Furthermore, content of the compound 3 in the liquid composition is, in a condensed product obtained from the compound 1 and the compound 2, preferably 25% by mass or higher, more preferably 35% by mass or higher, even more preferably 40% by mass or higher, even more preferably 45% by mass or higher, even more preferably 50% by mass or higher, and even more preferably 55% by mass or higher. The content of the compound 3 is the condensed product is 100% by mass or lower, and from the viewpoint of easy production from the compound 1 and the compound 2, it is preferably 75% by mass or lower and more preferably 60% by mass or lower.

The present invention relates to a method for producing the compound 3 by reacting the compound 1 and the compound 2 in water in the presence of alkyl diethanolamine, in which the molar feed ratio of the compound 1 to the compound 2 is, in terms of the compound 2/the compound 1, 0.3 or higher and 1.7 or lower, the reaction pH is 10.0 or higher, and the reaction temperature is 15°C or higher and 50°C or lower. Because the production method of the present invention is performed at a pH of 10.0 or higher until the end of the reaction, a reaction product in liquid form containing the compound 3 and water and having a pH of 10.0 or higher is obtained.

The liquid composition obtained by the production method of the present invention has high content of the compound 3 represented by the formula (3). Upon the ring opening, the compound 3 becomes a compound having two hydroxyl groups, that is, 1,3-(hydroxymethyl)urea (it may also be described as N,N'-dimethylol urea). This compound can be used as a chelating agent which is blended in a cleansing formulation, for example, a chelating agent for a steel cleansing formulation. Because a liquid composition containing the compound 3 is a liquid composition which contains 1,3-(hydroxymethyl)urea in an equilibrium state, the liquid composition obtained by the production method of the present invention can be used as a steel cleansing agent, a fertilizer composition for plant, an additive for paper making, a finishing agent for fibers, a water-resistant agent, an additive for hydraulic composition, a raw material for paint and ink, a raw material for organic synthesis, a raw material for resin, or the like.

Because a liquid composition containing the compound 3 is a liquid composition which contains 1,3-(hydroxymethyl)urea in an equilibrium state, it can be directly used as a chelating agent of a steel cleansing agent. Examples of the steel cleansing agent include a cleansing agent containing an alkalifying agent, a surface active agent, and water. Examples of the alkalifying agent include hydroxide of an alkali metal such as sodium hydroxide or potassium hydroxide. Examples of the surface active agent include a non-ionic surface active agent obtained by adding alkylene oxide to alcohol having 8 to 22 carbon atoms. Furthermore, examples other components that are blended in a steel cleansing agent include an anti-foaming agent, a dispersing agent such as polyacrylic acid, and a thickening agent. From the viewpoint of the property of cleansing oils such as press oil adhered to steel, in the liquid composition obtained by the present invention, the compound 3 is blended to have an amount of 0.01% by mass or higher or 0.1% by mass or higher, and 10% by mass or lower or 5.0% by mass or lower in a steel cleansing agent. Further, from the viewpoint of the property of cleansing oils such as press oil adhered to steel, the steel cleansing agent has a pH of 12 or higher, preferably 13 or higher, and more preferably 14 or higher at 20°C.

The process for which the steel cleansing agent using the liquid composition obtained by the production method of the present invention is used is a cleansing process, and examples thereof include continuous cleansing, that is, impregnation cleansing, spray cleansing, brush cleansing, and electrolytic cleansing. According to the process, contamination by oils such as press oil and solid contamination such as iron powder can be washed and removed. The steel cleansing agent can be used for a cleansing process including impregnation and electrolytic cleansing, and it can be preferably applied for a case where a pressed steel plate is passed through a roll in an alkali impregnation cleaning bath and an alkali electrolytic cleaning bath.

Hereinbelow, details of the storage solution of the present invention is explained.

Although the reason for exhibiting the effect of the present invention is not clear, it is speculated as follows.

Because dimethylol urea having an open-ring structure of tetrahydro-4H-1,3,5-oxadiazin-4-one [hereinbelow, also referred to as the compound 3] has two hydroxyl groups, it is believed that a cross-linking reaction between dimethylol ureas or between a dimethylol urea and a condensed product of urea and aldehyde can easily occur and thus insoluble white precipitations are yielded due to polymerization during storage. Structure of the compound 3 has a cyclic structure in which two hydroxyl groups of dimethylol urea are dehydrated and condensed to each other. As such, excellent storage stability is obtained due to low reactivity. In an aqueous solution, dimethylol urea and the compound 3 are in an equilibrium state. Thus, it is believed that, since it may easily have the structure of the compound 3 than the structure of dimethylol urea in a region of pH 9.5 or higher and 12.5 or lower and pH change of the liquid composition is suppressed by the pH buffering activity of alkanolamine, the cross-linking reaction of the aforementioned condensed product is suppressed and the storage stability of the liquid composition is enhanced.

### <Liquid Composition>

The storage solution of a liquid composition according to the present invention contains tetrahydro-4H-1,3,5-oxadiazin-4-one (the compound 3), alkanolamine, and water.

The compound 3 can be obtained as a condensed product of the compound 1 represented by the above formula (1) and the compound 2 represented by the above formula (2). The compound 3 is represented by the above formula (3).

According to the present invention, a condensed product, other than tetrahydro-4H-1,3,5-oxadiazin-4-one, of a compound represented by the formula (1) and a compound represented by the formula (2) may be added to the liquid composition.
Further, the liquid composition may contain a condensed product, other than the compound 3, of a compound represented by the formula (1) and a compound represented by the formula (2). Examples of a condensed product other than the compound 3 include a compound in which two molecules of dimethylol urea are dehydrated and condensed to form a ring. The structures of those compounds are expressed by the following general formula (4) and (5).

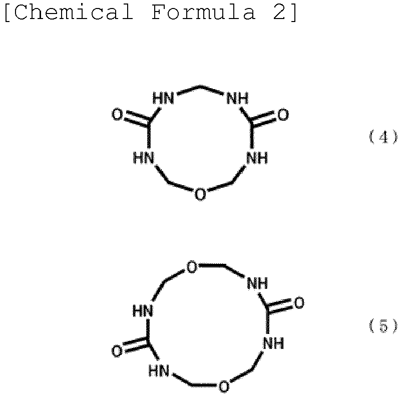

The content of the compound 3 in a condensed product of a compound represented by the formula (1) and a compound represented by the formula (2) is preferably 25% by mass or higher and more preferably 30% by mass or higher in the condensed product from the viewpoint of enhancing the storage stability. The content of the compound 3 is 100% by mass or lower in the condensed product, and from the viewpoint of having easy production from the compound 1 and the compound 2, it is preferably 75% by mass or lower and more preferably 60% by mass or lower.

The content of the compound 3 in a liquid composition according to the present invention is the same as that of the method for producing a liquid composition described above.

Total content of a condensed product of a compound represented by the formula (1) and a compound represented by the formula (2) in the liquid composition according to the present invention is, from the viewpoint of enhancing the blending property at the time of using a liquid composition, preferably 2.0% by mass or higher, more preferably 5.0% by mass or higher, and even more preferably 10% by mass or higher. From the viewpoint of enhancing the storage stability, it is preferably 30% by mass or lower, more preferably 20% by mass or lower, and even more preferably 15% by mass or lower.

As alkanolamine, alkanolamine having an active hydrogen in the nitrogen atom such as monoethanolamine and diethanolamine, and triethanolamine can be used. Furthermore, alkanolamine having an alkyl group with 1 to 3 carbon atoms can be used, and methyl diethanolamine, ethyl diethanolamine, propyl diethanolamine, dimethyl ethanolamine, or the like can be used. From the viewpoint of enhancing the storage stability of the liquid composition, monoethanolamine, diethanolamine, triethanolamine, and methyl diethanolamine are preferable. Diethanolamine, triethanolamine, and methyl diethanolamine are more preferable. From the viewpoint of having high pH buffering activity and suppressing pH change, methyl diethanolamine is even more preferable.

The content of alkanolamine in the liquid composition of the present invention is, from the viewpoint of enhancing the storage stability, preferably 0.05% by mass or higher, more preferably 0.1% by mass or higher, and even more preferably 0.3% by mass or higher. From the viewpoint of enhancing the storage stability, it is preferably 20% by mass or lower and more preferably 10% by mass or lower. Alkanolamine is preferably used as a pH controlling agent of the liquid composition. Further, when alkanolamine is used for producing a condensed product containing the compound 3 described below, it is preferably 0.1% by mass or higher, more preferably 0.3% by mass or higher, and even more preferably 0.5% by mass or higher. Furthermore, it is preferably 5.0% by mass or lower, more preferably 1.0% by mass or lower, and even more preferably 0.6% by mass or lower.

The mass ratio of alkanolamine to the compound 3 (alkanolamine/the compound 3) is, from the viewpoint of enhancing the storage stability, preferably 0.005 or higher, more preferably 0.010 or higher, and even more preferably 0.013 or higher. Furthermore, it is preferably 4.5 or lower, more preferably 2.5 or lower, and even more preferably 2.0 or lower. Further, when alkanolamine is used for producing a condensed product containing the compound 3 described below, it is preferably 1.000 or lower, more preferably 0.50 or lower, even more preferably 0.25 or lower, and even more preferably 0.18 or lower.

The content of water in the liquid composition is 70% by mass or higher, and from the viewpoint of enhancing storage stability, it is preferably 75% by mass or higher, and more preferably 80% by mass or higher. Furthermore, it is preferably 95% by mass or lower, more preferably 90% by mass or lower, and even more preferably 88% by mass or lower.

The content of a solid matter in the liquid composition is, from the viewpoint of enhancing the blending property at the time of using a liquid composition, preferably 5% by mass or higher, more preferably 10% by mass or higher, and even more preferably 12% by mass or higher. From the viewpoint of enhancing the storage stability, it is preferably 30% by mass or lower, more preferably 25% by mass or lower, and even more preferably 20% by mass or lower.

In the present invention, from the viewpoint of enhancing storage stability by maintaining the pH at 10.0 or higher and 12.5 or lower, the pH of the liquid composition is preferably 12.0 or lower and more preferably 11.5 or lower. The storage solution preferably has a pH of 10.0 or higher at 20°C.

Within a range in which the effect of the present invention is not inhibited, a polar solvent (for example, methanol and ethanol) other than water can be contained in the liquid composition. The ratio of water in the solvent is preferably 50% by mass or higher, more preferably 70% by mass or higher, 90% by mass or higher, or 100% by mass. Further, within a range in which the effect of the present invention is not inhibited, an anti-foaming agent or the like may be contained.

### <Method for Producing Liquid Composition according to Storage Method>

With regard to the liquid composition, a method of controlling a pH by mixing commercially available methylol urea with water and adding a pH controlling agent can be mentioned. As a pH controlling agent, alkanolamine is used from the viewpoint of suppressing pH change. As another pH controlling agent, inorganic acid, organic acid, inorganic alkali, and organic alkali may be used in combination.

Further, the liquid composition is obtained by the aforementioned method for producing a liquid composition. However, from the viewpoint of suppressing pH decrease during a reaction when a condensed product containing the compound 3 is produced by reacting the compound 1 and the compound 2 using water as a solvent, a method of obtaining the liquid composition of the present invention by performing the reaction at alkali condition in the presence of alkanolamine can be mentioned.

The molar feed ratio of the compound 2 to the compound 1 for the production method is the same as that of the method for producing a liquid composition described above. Furthermore, it is 1.7 or lower in terms of the compound 2/the compound 1.

The total feed amount of the compound 1 and the compound 2 is the same as that of the method for producing a liquid composition described above.

The feed amount of the compound 1 is the same as that of the method for producing a liquid composition described above.

The feed amount of the compound 2 is the same as that of the method for producing a liquid composition described above.

The feed amount of water is the same as that of the method for producing a liquid composition described above.

The feed amount of alkanolamine is, from the viewpoint of enhancing the yield of the compound 3, preferably 0.1 part by mass or higher, more preferably 0.3 part by mass or higher, and even more preferably 0.5 part by mass or higher relative to 100 parts by mass of the total amount of raw materials (the compound 1, the compound 2, alkanolamine, and water). Furthermore, from the viewpoint of saturated pH buffering activity, it is preferably 5.0 parts by mass or lower, more preferably 1.0 part by mass or lower, and even more preferably 0.6 part by mass or lower.

The molar feed ratio of alkanolamine to the total amount of the compound 1 and the compound 2 [alkanolamine/(total of the compound 1 and the compound 2)] is, from the viewpoint of enhancing the yield of the compound 3, preferably 0.005 or higher, more preferably 0.010 or higher, and even more preferably 0.013 or higher. It is also preferably 0.100 or lower, more preferably 0.050 or lower, even more preferably 0.025 or lower, and even more preferably 0.018 or lower.

The reaction temperature is the same as that of the method for producing a liquid composition as described above.

The reaction pH indicates a pH from the start to the end of the reaction. With regard to the reaction between the compound 1 and the compound 2, there is a tendency that a pH decreases in accordance with a progress of the reaction. From the viewpoint of suppressing pH decrease by the pH buffering activity, it is preferable that the reaction between the compound 1 and the compound 2 is carried out in the presence of alkanolamine. The reaction pH for the production method is, from the viewpoint of enhancing storage stability of the liquid composition, 10.0 or higher. From the viewpoint of enhancing the blending property at the time of using the liquid composition, the pH is preferably 13.0 or lower, more preferably 12.0 or lower, and even more preferably 11.5 or lower. From the viewpoint of storage stability of the liquid composition, it is preferably 10.5 or higher and more preferably 11.0 or higher. It is also preferably 13.0 or lower, more preferably 12.5 or lower, even more preferably 12.0 or lower, and even more preferably 11.5 or lower. From the viewpoint of enhancing the storage stability of the liquid composition, the pH is maintained within the aforementioned range during the reaction. It is also preferable that the pH at the end of the reaction is within the range of the present invention. Meanwhile, the reaction pH indicates a pH of a reaction system, that is, a pH of a mixture containing water, the compound 1, the compound 2, and alkanolamine.

The reaction time is, from the viewpoint of enhancing the yield of the condensed product of the compound 2 and the compound 1, preferably 1.5 hours or longer. More preferably, it is 2 hours or longer. Furthermore, from the viewpoint of enhancing the yield of the compound 3, the reaction time is preferably 4.5 hours or shorter, and more preferably 3 hours or shorter.

Considering the total of the raw materials (the compound 1, the compound 2, alkanolamine, and water), the time points for starting the reaction and ending the reaction are the same as those of the method for producing a liquid composition described above.

Considering alkanolamine, the method for adding the compound 2 is also the same as that of the method for producing a liquid composition described above.

Combined use of a reaction solvent is also the same as that of the method for producing a liquid composition described above.

It is also possible to add further alkanolamine to a reaction mixture which is obtained as described above.

### <Method for Storing Liquid Composition and Its Application>

With regard to the liquid composition, from the viewpoint of extending the storage period, the storage temperature is preferably 60°C or lower and more preferably 50°C or lower. It is also preferably 0°C or higher. A container for storage is not particularly limited, and those made of glass, polypropylene, or the like can be used.

The liquid composition of the present invention contains the compound 3 represented by the formula (3). Upon the ring opening, the compound 3 becomes a compound having two hydroxyl groups, that is, 1,3-(hydroxymethyl)urea (it may also be described as N,N'-dimethylol urea). The liquid composition of the present invention has the same uses as those of the aforementioned method for producing a liquid composition.

Hereinbelow, aspects of the present invention are exemplified.

### <1>

A method for producing, by reacting a compound 1 represented by the following formula (1) and a compound 2 represented by the following formula (2) in water, a liquid composition with a pH of 10.0 or higher containing a compound 3 represented by the following formula (3) and water, which is a method for producing a liquid composition containing tetrahydro-4H-1,3,5,-oxadiazin-4-one, in which
the molar feed ratio of the compound 2 to the compound 1, that is, the compound 2/the compound 1, is 0.3 or higher and 1.7 or lower, and
the reaction between the compound 1 and the compound 2 is performed at 15°C or higher and 50°C or lower and a pH of 10.0 or higher in the presence of alkyl diethanolamine.

### <2>

The production method described in <1>, in which the molar feed ratio of alkyl diethanolamine to the total of the compound 1 and the compound 2 (alkyl diethanolamine/(total of the compound 1 and the compound 2) is 0.005 or higher and 0.100 or lower, more preferably 0.010 or higher, and even more preferably 0.013 or higher, and also more preferably 0.050 or lower, even more preferably 0.025 or lower, and even more preferably 0.018 or lower.

### <3>

The production method described in <1> or <2>, in which the reaction between the compound 1 and the compound 2 is performed by adding the compound 2 to a mixture containing the compound 1, alkyl diethanolamine, and water.

### <4>

The production method described in any of <1> to <3>, in which the total feed amount of the compound 1 and the compound 2 is preferably 3.0 parts by mass or higher and 25 parts by mass or lower, more preferably 6.0 parts by mass or higher, and even more preferably 11 parts by mass or higher and also more preferably 20 parts by mass or lower, and even more preferably 14 parts by mass or lower relative to 100 parts by mass of the total amount of the compound 1, the compound 2, alkyl diethanolamine, and water.

### <5>

The production method described in any of <1> to <4>, in which the feed amount of alkyl diethanolamine is preferably 0.1 part by mass or higher and 5.0 parts by mass or lower, more preferably 0.3 part by mass or higher, and even more preferably 0.5 part by mass or higher and also more preferably 1.0 part by mass or lower and even more preferably 0.6 part by mass or lower relative to 100 parts by mass of the total amount of the compound 1, the compound 2, alkyl diethanolamine, and water.

### <6>

The production method described in any of <1> to <5>, in which the feed amount of water is preferably 70 parts by mass or higher and 95 parts by mass or lower, more preferably 75 parts by mass or higher, and even more preferably 80 parts by mass or higher and also more preferably 90 parts by mass or lower, and even more preferably 88 parts by mass or lower relative to 100 parts by mass of the total amount of the compound 1, the compound 2, alkyl diethanolamine, and water, and for a case of using a raw material other than water in an aqueous solution form, the amount of water taken in by the aqueous solution to a reaction system is also included in the amount of water as a reaction solvent.

### <7>

The production method described in any of <1> to <6>, in which the reaction time is preferably 1 hour or longer and 4.5 hours or shorter, more preferably 1.5 hours or longer, and even more preferably 2 hours or longer and also more preferably 3 hours or shorter.

### <8>

The production method described in any of <1> to <7>, in which the content of the compound 3 in the obtained liquid composition is preferably 1.4% by mass or higher and 30% by mass or lower, more preferably 2.0% by mass or higher, even more preferably 2.5% by mass or higher, and even more preferably 3.2% by mass or higher and also more preferably 15% by mass or lower, even more preferably 10% by mass or lower, and even more preferably 8.0% by mass or lower.

### <9>

The production method described in any of <1> to <8>, in which alkyl diethanolamine is preferably alkyl diethanolamine having an alkyl group with 1 to 3 carbon atoms, more preferably alkyl diethanolamine having an alkyl group with 1 carbon atom, and even more preferably N-methyl diethanolamine.

### <10>

A storage solution of a liquid composition obtained by the production method described in any of <1> to <9>, in which the compound 3, alkyl diethanolamine, and water are contained, the content of water in the storage solution is preferably 70% by mass or higher, more preferably 75% by mass or higher, and even more preferably 80% by mass or higher and also preferably 95% by mass or lower, more preferably 90% by mass or lower, and even more preferably 88% by mass or lower, and the pH is 10.0 or higher and 12.5 or lower, preferably 12.0 or lower, and more preferably 11.5 or lower.

### <11>

The storage solution described in <10>, in which the pH at 20°C is 10.0 or higher, or preferably 10.0 or higher and 12.5 or lower, more preferably 12.0 or lower, and even more preferably 11.5 or lower.

### EXAMPLES

The following examples are given to describe the embodiments of the present invention.

Examples and Comparative Examples regarding the method for producing liquid composition

### <Preparation of Composition A>

450 g (7.50 mol) of urea (the compound 1) and 3588 g of distilled water were added to a three-neck flask with a volume of 10 L. By using a stirring wing (three wings, diameter of 70 mm), it was mixed for 10 minutes at 200 rpm. After that, 27.0 g (0.23 mol) of N-methyl diethanolamine was added, the pH was adjusted to 11.3, and the temperature was raised to 40°C. Subsequently, 305 g (3.75 mol as formaldehyde (the compound 2)) of 37% formalin was added dropwise thereto over 90 minutes. After the dropwise addition was completed, it was aged by continuing the stirring for 1 hour. After cooling it to room temperature, the composition A in a liquid form with the solid content of 13.3% by mass was obtained. At that time, the pH of the composition A was 11.0 and the content of the compound 2 was 1% by mass or lower relative to the feed amount of the raw materials (the compound 1, the compound 2, alkyl diethanolamine, and water; the same is applied to the followings). The molar feed ratio of the compound 2 to the compound 1, that is, the compound 2/the compound 1, was 0.5, and the total amount of the compound 1 and the compound 2 was 12.87 parts by mass, water was 86.5 parts by mass, and N-methyl diethanolamine was 0.63 part by mass relative to 100 parts by mass of the fed raw materials. Meanwhile, the pH at the reaction start point described in the table indicates the pH of a reaction system when the compound 1 and the compound 2 are initially brought into contact with each other, and the pH at the reaction end point indicates the pH of a reaction system after dropwise addition for 90 minutes and further aging for 1 hour, which corresponds to pH of the obtained composition in this example (ditto for the followings). The amount of the compound 2 was measured after aging for 30 minutes and 1 hour. The amount of the compound 2 was 0.8% by mass after 30 minutes and 0.6% by mass after 1 hour relative to the total raw materials. It was confirmed that the reduced amount of the compound 2 for 30 minutes was 0.3% by mass or lower relative to the total raw materials. It was determined that the reaction was completed at the time point of aging for 1 hour.

### <Preparation of Compositions B to D and a to d>

The compositions were prepared in the same manner as the composition A except that the amount of the compound 1, the compound 2, and alkyl diethanolamine was changed to those described in Table 1. Meanwhile, the compound D was prepared by adding formalin in advance to a flask and mixing therein in the same manner as urea (the feeding method in the table is described as "en masse"). For the preparation of the composition D, it was confirmed that the reduced amount of the compound 2 for 30 minutes at the reaction end point was 0.3% by mass or lower relative to the total raw materials (the compound 1, the compound 2, alkyl diethanolamine, and water).

### <Preparation of Compositions E, F and e to g>

The compositions were prepared in the same manner as the composition B except that the reaction temperature was changed to the temperature of Table 2. In Table 2, conditions and results of the composition B are also described.

### <Preparation of Compositions h to j>

The compositions were prepared in the same manner as the compound B except that N-methyl diethanolamine was changed to the compounds of Table 3. In Table 3, conditions and results of the composition B are also described.

### <Preparation of Composition k>

By dissolving 13.5 g of 1,3-bis(hydroxymethyl)urea (commercially available product, a reagent manufactured by Wako Pure Chemical Industries, Ltd.) in a powder form in 86.5 g of distilled water, the composition Q with the solid content of 13.5% by mass (pH 7.4 at 20°C) was produced.

The content of the compound 3 in the commercially available powder was 46.4% by mass. Meanwhile, in the composition Q, 1,3-bis(hydroxymethyl)urea is in an equilibrium relationship with the structure of the compound 3.

The following compounds were used for each compound in Tables 1 to 3.
- urea: manufactured by Wako Pure Chemical Industries, Ltd., reagent
- formalin: 37% by mass aqueous solution, manufactured by Wako Pure Chemical Industries, Ltd., reagent
- N-methyl diethanolamine: manufactured by Nippon Nyukazai Co., Ltd., aminoalcohol MDA
- triethanolamine: manufactured by Wako Pure Chemical Industries, Ltd., reagent
- diethanolamine: manufactured by Wako Pure Chemical Industries, Ltd., reagent
- monoethanolamine: manufactured by Wako Pure Chemical Industries, Ltd., reagent

### <Content of Condensed Product of Compound 2 and Compound 1>

According to Karl Fisher method, the content of water in the liquid composition was obtained, and the mass of the components other than water was determined as the solid content. The content of urea in the liquid composition was obtained by liquid chromatography. In the same manner, the content of formaldehyde in the liquid composition was obtained by liquid chromatography. Then, the amount obtained by subtracting the contents of urea and formaldehyde from the solid content was determined as the content of a condensed product of the compound 2 and the compound 1 in the liquid composition.

### <Measurement of Content of Compound 3 in Condensed Product>

A part of the obtained composition (about 20 g) was prepared as powder by freeze drying. Subsequently, by using 1% by mass aqueous solution of the powder, the content of tetrahydro-4H-1,3,5-oxadiazin-4-one, which is the compound 3, in the powder was obtained by liquid chromatography mass spectroscopy (LC/MS). Specifically, from the area of 120.0 (m/z) peak of the compound 3 by mass spectroscopy and the area of each peak of the liquid chromatography which corresponds to 192.1, 221.9, 285.9 and 316.0 (m/z) peaks of the condensed product other than the compound 3, the content of the compound 3 in the condensed product was obtained. The content of the compound 3 in the condensed product as described in the tables corresponds to a ratio of the peak area of the aforementioned monomer to the total peak area of the aforementioned monomer and multimer in the liquid composition. The content of the compound 3 in the liquid composition was obtained from the solid matter amount in the liquid composition, the content of the condensed product in the solid matter, and the content of the compound 3 in the condensed product.

### ⊙ Conditions for liquid chromatography mass analysis

Column: Imtakt Union UK-Amino HT (length of 150 mm and inner diameter of 2 mm) 3 µ
Column temperature: 40°C
Mobile phase: A 0.01 mol/L aqueous solution of ammonium acetate, C acetonitrile (gradient elution method)

### <Storage Stability Test>

The obtained composition was added to a 500 ml polypropylene bottle and stored at 40°C. One day later, one week later, one month later, or three months later after starting the storage, the outer appearance was observed with a naked eye. When there is no precipitation or turbidity in the composition, it was evaluated as A. When there is turbidity, it was evaluated as B. When there is a precipitation, it was evaluated as C.

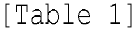

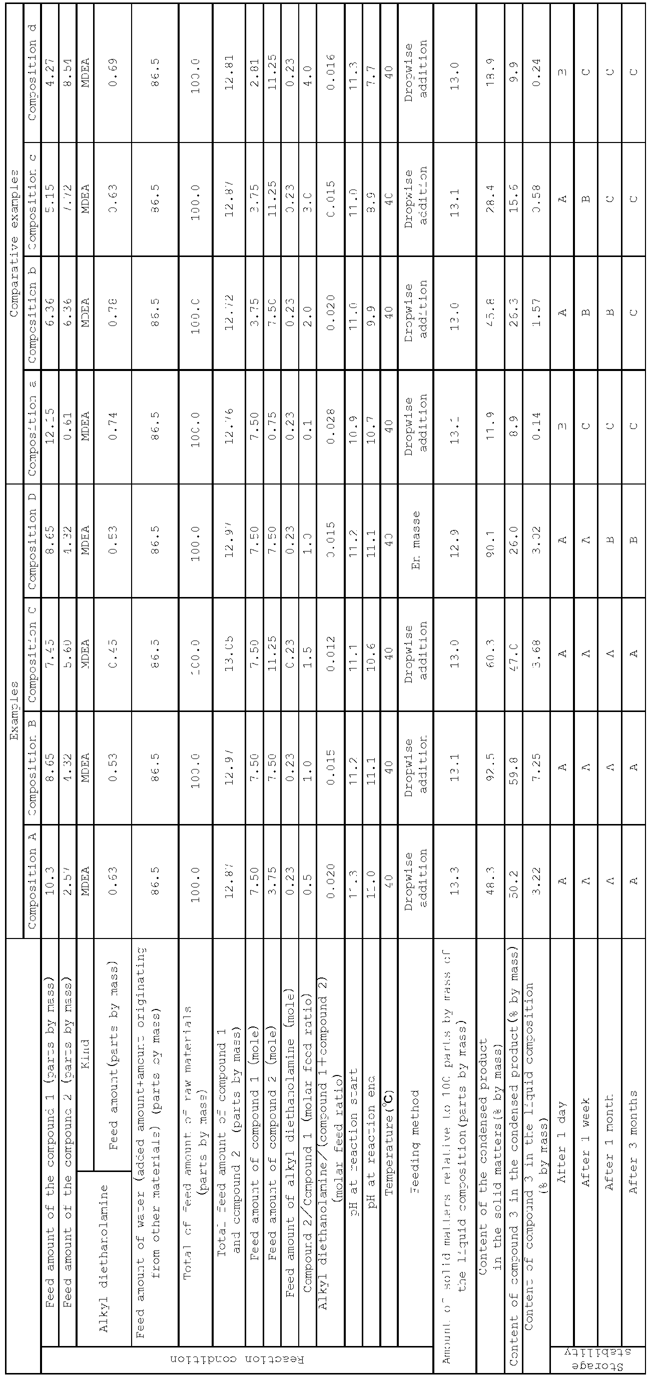

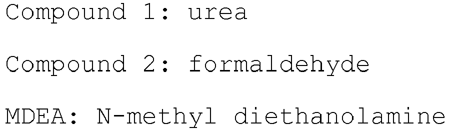

Within a range in which the molar ratio of the compound 2 to the compound 1, that is, the compound 2/the compound 1, is 0.3 or higher and 1.7 or lower, it can be seen that the compound 3 has excellent yield and storage stability.

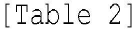

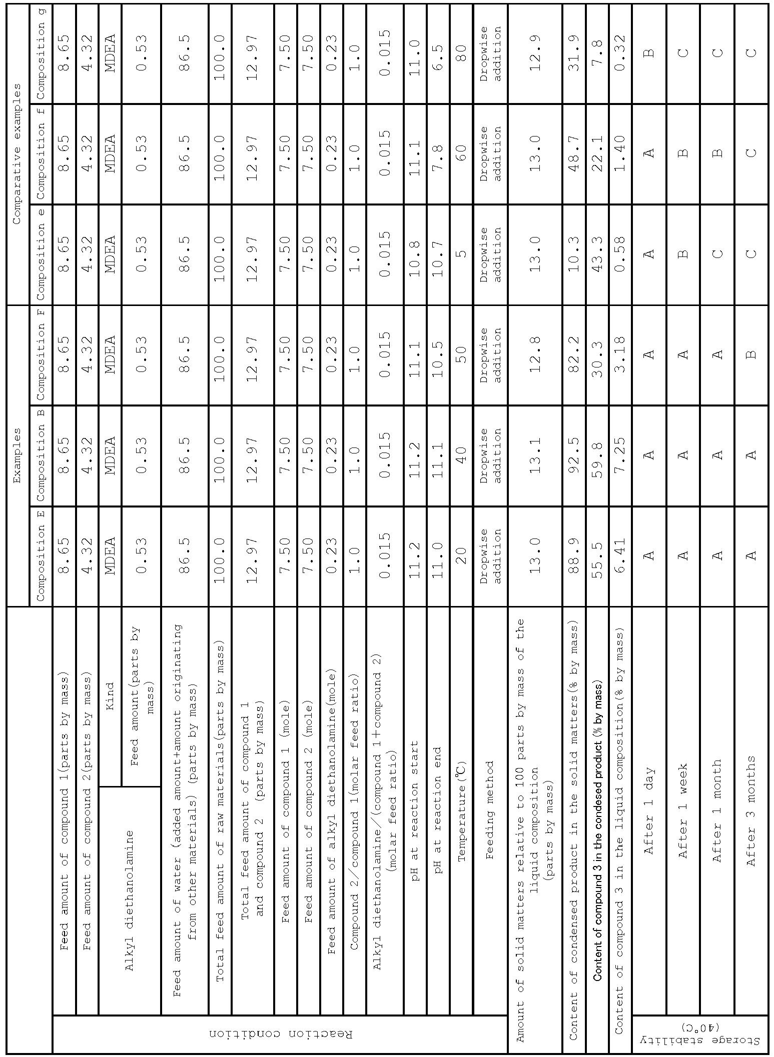

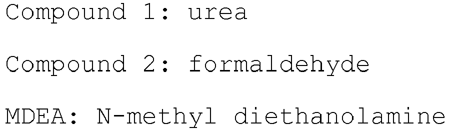

Within a range in which the reaction temperature is 15°C or higher and 50°C or lower, it can be seen that the compound 3 has excellent yield and storage stability.

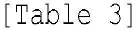

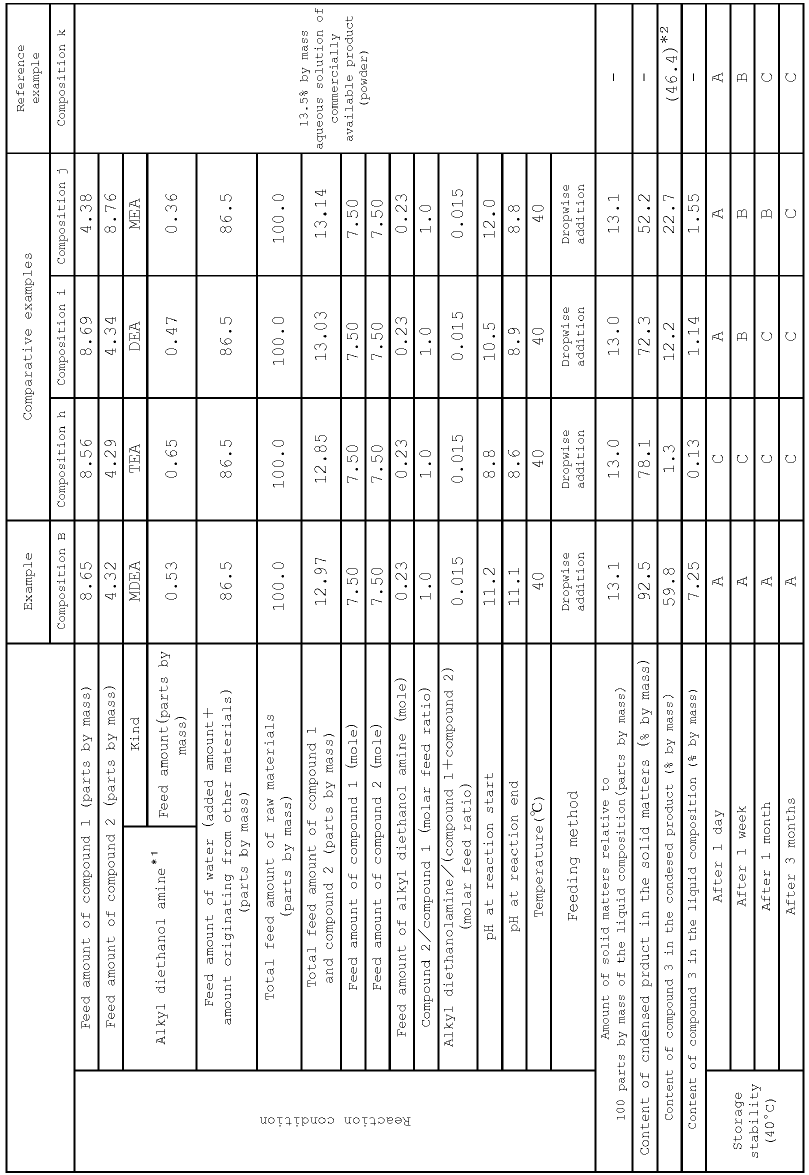

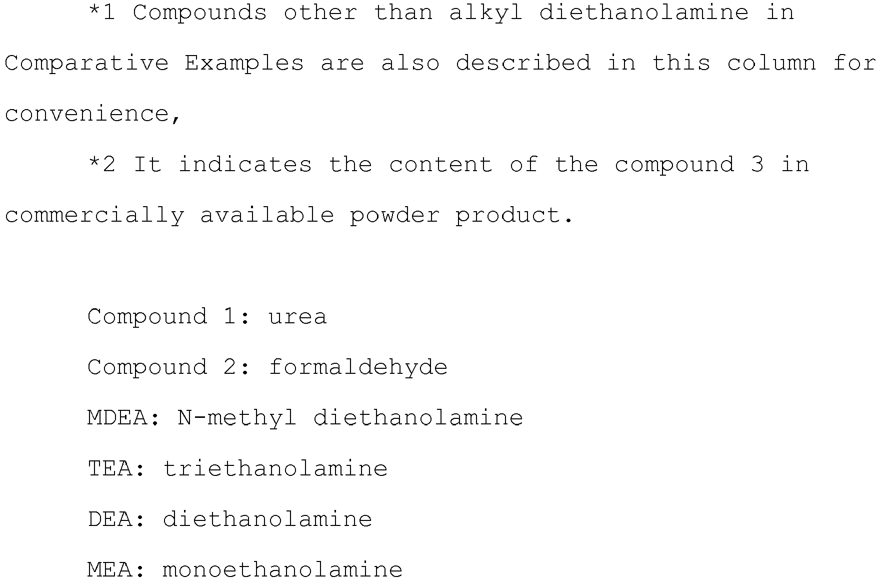

It can be seen that the compound 3 has excellent yield and storage stability when alkyl diethanolamine is used and pH is 10.0 or higher at the time of the reaction.

It can also be seen that the content of the compound 3 as a commercially available powder product described in the reference example is 46.4% by mass and a liquid composition obtained by using a commercially available powder product has pH of 7.4 and poor storage stability.

### Test Examples

A steel cleansing agent was prepared by using the components that are described in Table 4, and the steel cleansing property was evaluated.

### <Preparation of Steel Cleansing Agent>

### (1) Preparation of Cleansing Agent A

In terms of the ratio in a final cleansing agent, 1.0% by mass of polyoxyethylene (added 2 mol on average)-2-ethyl hexyl ether, 5.0% by mass of sodium polyacrylate (weight average molecular weight of 6000), 13.0% by mass of the composition B (corresponding to 2.0% by mass of the compound 3), and 61.0% by mass of water were admixed with each other to prepare an aqueous solution. Subsequently, in terms of the ratio in a final cleansing agent, 20.0% by mass of sodium hydroxide was added to the aqueous solution and mixed therein to obtain the cleansing agent A.

### (2) Preparation of Comparative Cleansing Agent B

The cleansing agent B was prepared in the same manner as the cleansing agent A except that sodium ethylenediamine tetraacetate, which is known as a chelating agent, is used in an amount of 2.0% by mass instead of the composition B and the water ratio is set at 72.0% by mass.

### (3) Preparation of Comparative Cleansing Agent C

The comparative cleansing agent C was prepared in the same manner as the cleansing agent A except that the composition B and sodium ethylenediamine tetraacetate are not added and the water ratio is set at 74.0% by mass.

### <Evaluation of Cleansing Property>

A steel piece (10 cm x 15 cm) adhered with synthetic ester-based press oil was washed by immersing in an aqueous solution of the cleansing agent which has been diluted to 20 times (temperature of the cleansing solution: 60°C, and time for immersion: 1 second). Subsequently, after performing electrolytic cleansing (temperature of the cleansing solution: 60°C, time for electrolysis: 1 second (0.5 second for each of +/-), and current density of 10 A/dm² (initial minus electrode was used as a steel for cleansing)), it was washed with a sufficient amount of running water. Then, the area of the steel surface wetted by water was measured and the cleansing property was evaluated. As the area wetted by water is closer to 100%, it indicates a higher cleansing property.

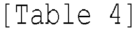

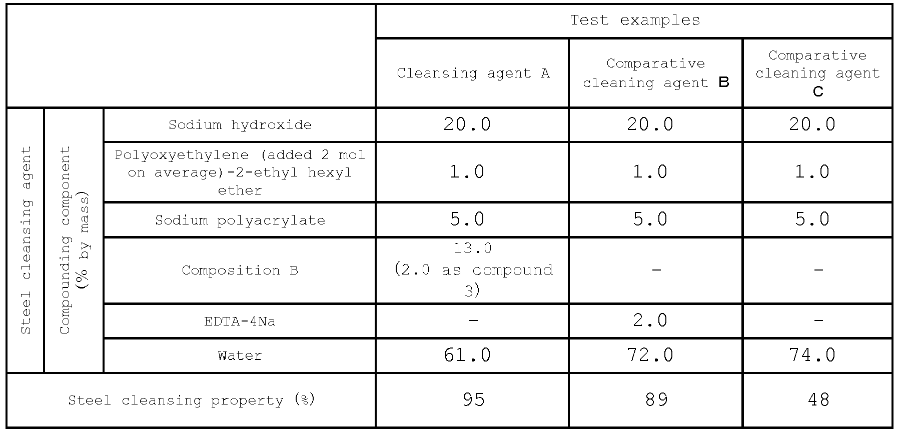

It can be seen that the cleansing agent A has better cleansing property than the comparative cleansing agents B and C, and the composition obtained by the production method of the present invention can be used as a chelating agent of a cleansing agent.

### Example and Comparative Example related to Method for Storing Liquid Composition

### <Preparation of Composition 2b>

By dissolving 13.0 g of 1,3-bis(hydroxymethyl)urea (commercially available product, a reagent manufactured by Wako Pure Chemical Industries, Ltd.) in a powder form in 87.0 g of distilled water, the composition 2b with a solid content of 13.0% by mass (pH 7.4 at 20°C) was produced.

The content of the compound 3 in the commercially available powder was 46.4% by mass. Meanwhile, in the composition 2b, 1,3-bis(hydroxymethyl)urea is in an equilibrium relationship with the structure of the compound 3.

### <Preparation of Compositions 2A to 2E and 2a>

The preparation was performed in the same manner as the composition 2b except that alkanolamine described in Table 6 is used in an amount described in Table 6.

### <Preparation of Composition 2c>

A liquid composition was prepared by performing the same preparation as the composition 2b followed by addition of 0.05 g of acetic acid.

### <Preparation of Composition 2d>

A liquid composition was prepared by performing the same preparation as the composition 2b followed by addition of 0.02 g of 48% aqueous solution of sodium hydroxide (0.01 g of sodium hydroxide).

### <Preparation of Composition 2I>

450 g (7.50 mol) of urea (the compound 1) and 3588 g of distilled water were added to a three-neck flask with volume of 10 L. By using a stirring wing (three wings, diameter of 70 mm), it was mixed for 10 minutes at 200 rpm. After that, 27.0 g (0.23 mol) of N-methyl diethanolamine was added, pH was adjusted to 11.3, and the temperature was raised to 40°C. Subsequently, 305 g (3.75 mol as formaldehyde (the compound 2)) of 37% formalin was added dropwise thereto over 90 minutes. After the dropwise addition is completed, it was aged by continuing the stirring for 1 hour. After cooling to room temperature, the composition 2I with a solid content of 13.3% by mass was obtained. At that time, pH of the composition 2I was 11.0 and the content of the compound 2 was 1% by mass or lower relative to the feed amount of the raw materials (the compound 1, the compound 2, alkyl diethanolamine, and water; ditto for the followings). The molar feed ratio of the compound 2 to the compound 1, that is, the compound 2/the compound 1, was 0.5, the total amount of the compound 1 and the compound 2 was 12.87 parts by mass relative to 100 parts by mass of the fed raw materials, water was 86.5 parts by mass, and N-methyl diethanolamine was 0.63 parts by mass. Meanwhile, the pH at reaction start point described in the table indicates pH of a reaction system when the compound 1 and the compound 2 are initially brought into contact with each other, and the pH at reaction end point indicates pH of a reaction system when the amount of the compound 2 is 0.3% by mass or lower relative to the total amount of the raw materials, and it corresponds to pH of the obtained composition in this example.

### <Preparation of Compositions 2F, 2G, 2H, 2J, and 2K>

The preparation was performed in the same manner as the composition 2I except that the amount of the compound 1, the compound 2, and methyl diethanolamine and reaction temperature are changed to those of Table 5. However, the composition 2F was prepared by adding in advance formalin to the flask followed by mixing in the same manner as urea.

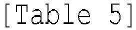

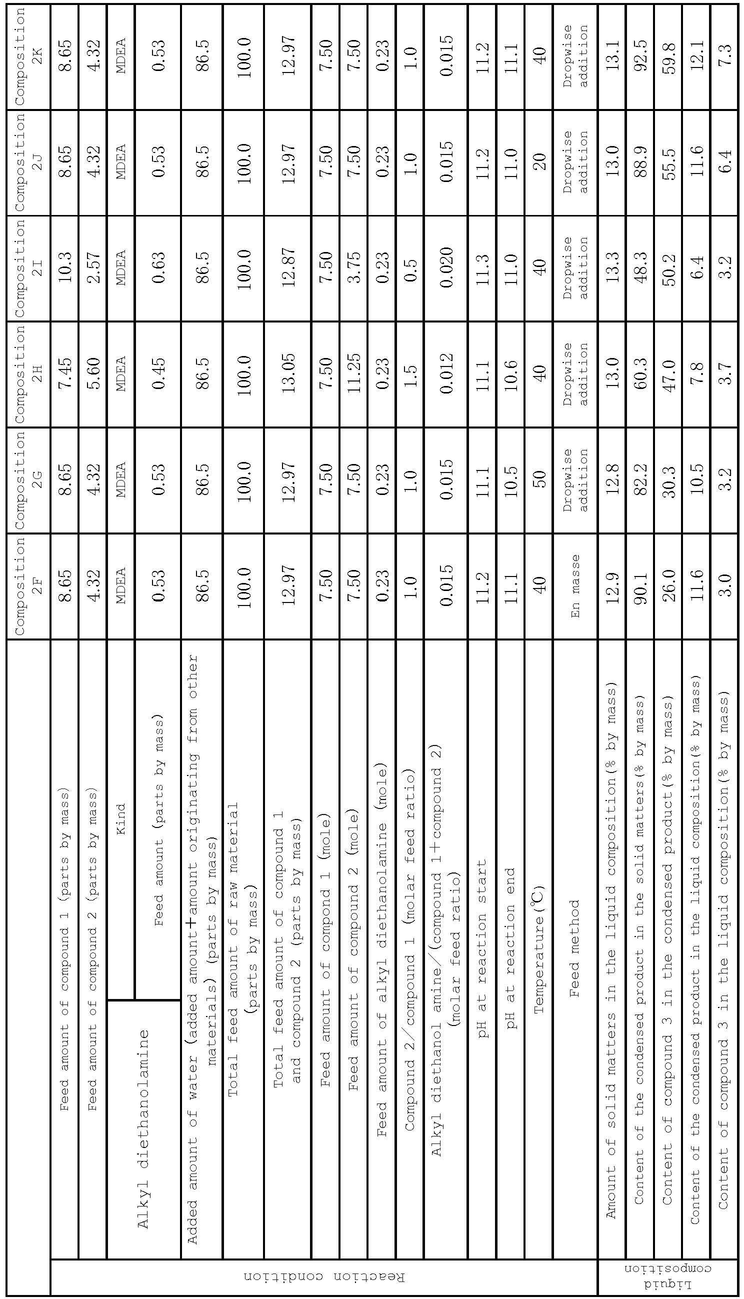

The following compounds were used for each compound of Table 5.
Compound 1: urea, manufactured by Wako Pure Chemical Industries, Ltd., reagent
Compound 2: formalin, 37% by mass aqueous solution, manufactured by Wako Pure Chemical Industries, Ltd., reagent
MDEA: N-methyl diethanolamine, manufactured by Nippon Nyukazai Co., Ltd., aminoalcohol MDA

Measurement of the content of a condensed product of the compound 2 and the compound 1, the content of the compound 3 in the condensed product, and conditions for liquid chromatography mass analysis were the same as those described above.

### <Compositions 2L to 2R and 2f>

The preparation was performed in the same manner as the composition 2b by using 1,3-bis(hydroxymethyl)urea (commercially available product, manufactured by Wako Pure Chemical Industries, Ltd., reagent) in a powder form in an amount described in Table 8, methyl diethanolamine, and distilled water.

The storage stability test was performed in the same manner as above. However, a test after three months was not performed.

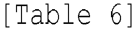

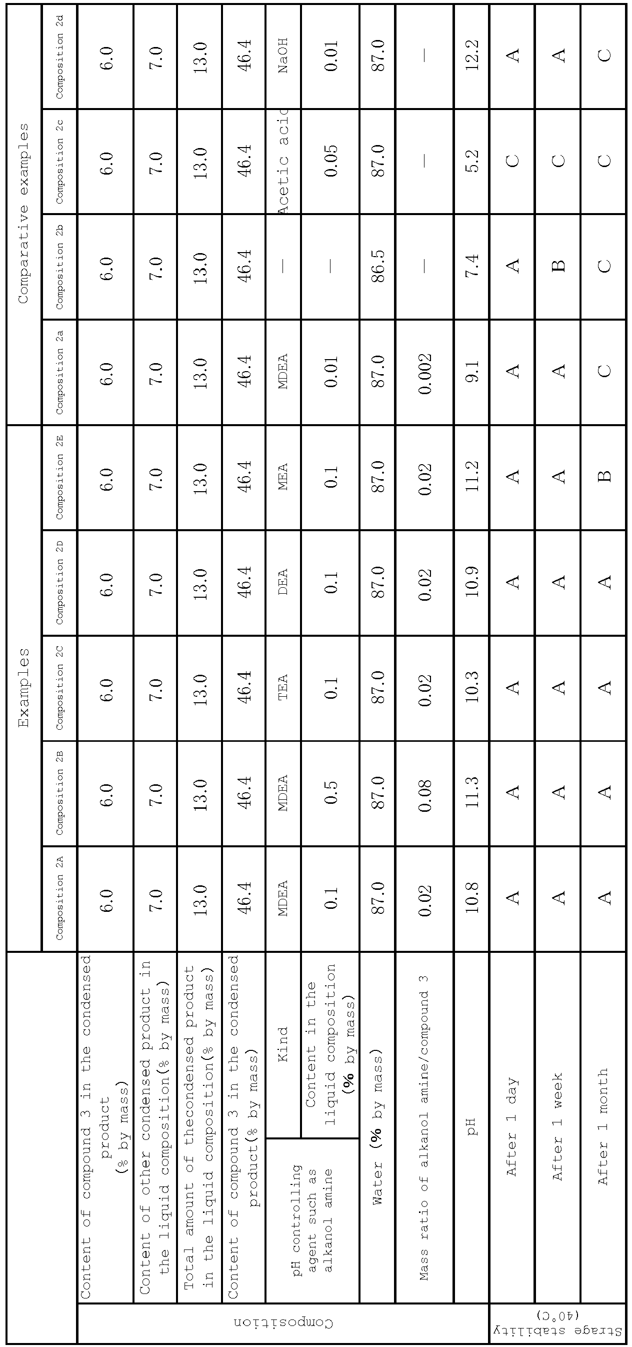

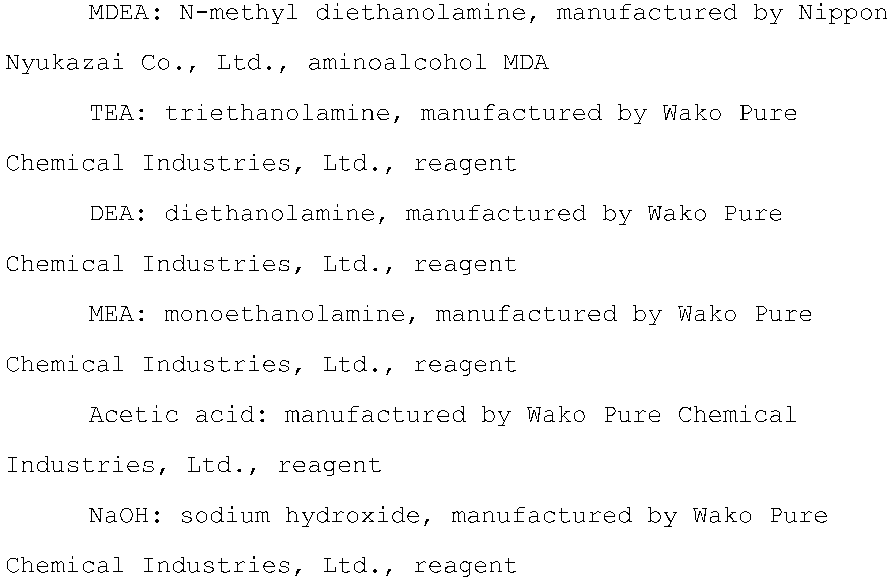

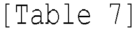

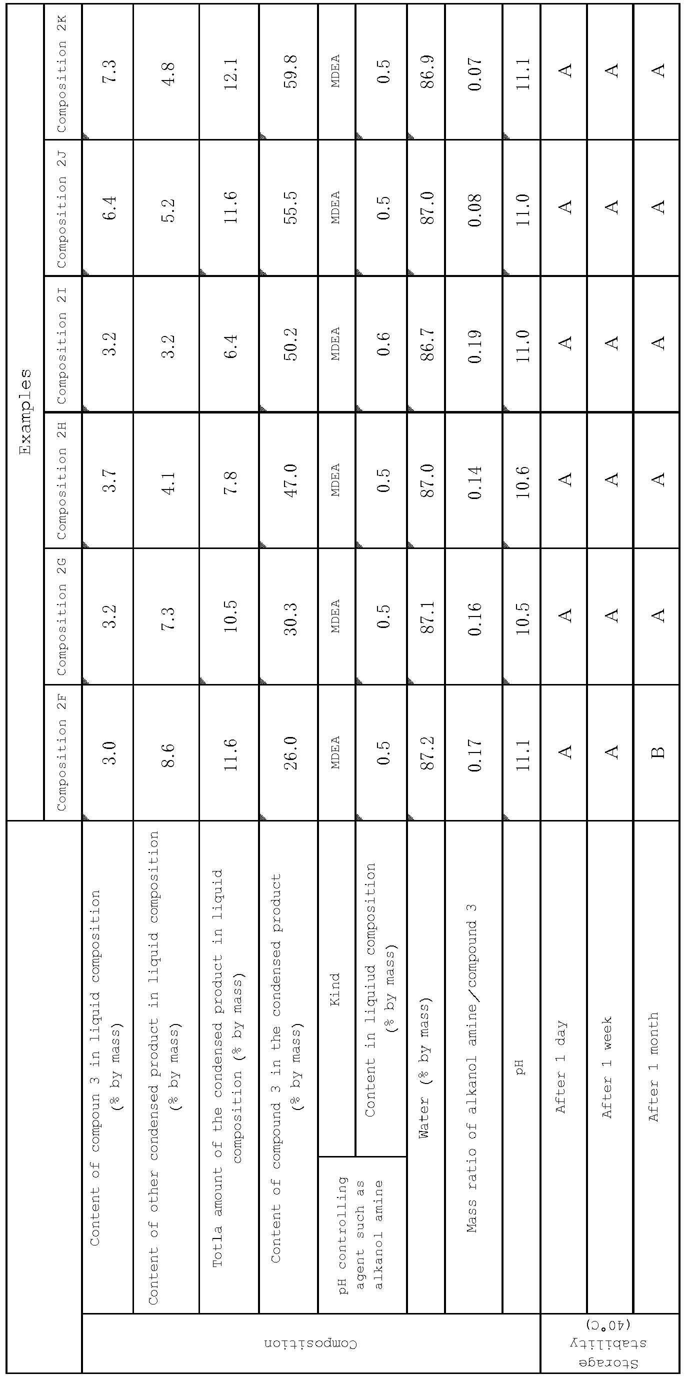

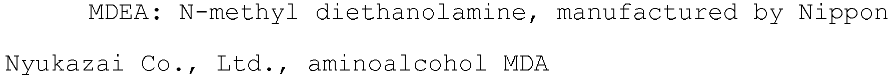

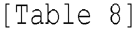

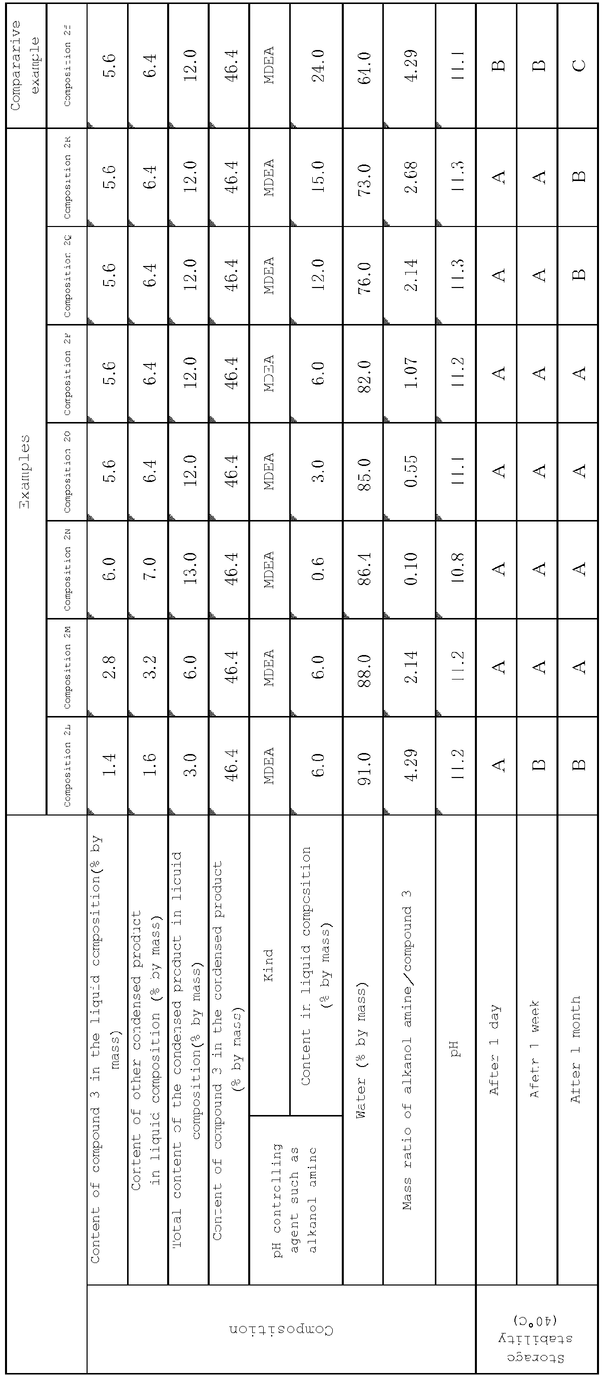

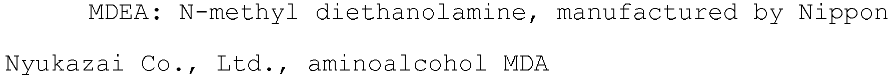

From Table 6 to Table 8, it can be seen that, when alkanolamine is contained, water content in the condensed product is 70% by mass or higher, and pH is 9.5 or higher and 12.5 or lower, excellent storage stability is exhibited.

## Claims

1. A method for producing, by reacting a compound 1 represented by the following formula (1) and a compound 2 represented by the following formula (2) in water, a liquid composition with a pH of 10.0 or higher containing a compound 3 represented by the following formula (3) and water, which is a method for producing a liquid composition containing tetrahydro-4H-1,3,5,-oxadiazin-4-one, wherein
the molar feed ratio of the compound 2 to the compound 1, that is, the compound 2/the compound 1, is 0.3 or higher and 1.7 or lower, and
the reaction between the compound 1 and the compound 2 is performed at 15°C or higher and 50°C or lower and a pH of 10.0 or higher in the presence of alkyl diethanolamine.

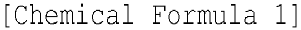

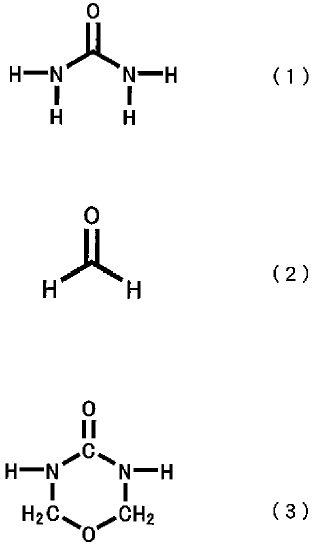

2. The method for producing a liquid composition according to claim 1, wherein the molar feed ratio of alkyl diethanolamine to the total amount of the compound 1 and the compound 2, alkyl diethanolamine/(total of the compound 1 and the compound 2), is 0.005 or higher and 0.10 or lower.

3. The method for producing a liquid composition according to claim 1 or 2, wherein the reaction between the compound 1 and the compound 2 is performed by adding the compound 2 to a mixture containing the compound 1, alkyl diethanolamine, and water.

4. The method for producing a liquid composition according to any of claims 1 to 3, wherein the total feed amount of the compound 1 and the compound 2 is 3.0 parts by mass or higher and 25 parts by mass or lower relative to 100 parts by mass of the total amount of the compound 1, the compound 2, alkyl diethanolamine, and water.

5. The method for producing a liquid composition according to any of claims 1 to 4, wherein the feed amount of alkyl diethanolamine is 0.1 part by mass or higher and 5.0 parts by mass or lower relative to 100 parts by mass of the total amount of the compound 1, the compound 2, alkyl diethanolamine, and water.

6. The method for producing a liquid composition according to any of claims 1 to 5, wherein the feed amount of water is 70 parts by mass or higher and 95 parts by mass or lower relative to 100 parts by mass of the total amount of the compound 1, the compound 2, alkyl diethanolamine, and water.

7. The method for producing a liquid composition according to any of claims 1 to 6, wherein the reaction time is 1 hour or longer and 4.5 hours or shorter.

8. The method for producing a liquid composition according to any of claims 1 to 7, wherein the content of the compound 3 in the obtained liquid composition is 1.4% by mass or higher and 30% by mass or lower.

9. The method for producing a liquid composition according to any of claims 1 to 8, wherein alkyl diethanolamine is N-methyl diethanolamine.

10. The method for producing a liquid composition according to any of claims 1 to 9, wherein the reaction between the compound 1 and the compound 2 is performed at a pH of 13.0 or lower.

11. A storage solution of a liquid composition obtained by the method for producing a liquid composition according to any of claims 1 to 10, the storage solution comprising the compound 3, alkyl diethanolamine, and water, wherein the content of water in the storage solution is 70% by mass or higher, and the pH is 10.0 or higher and 12.5 or lower.

12. The storage solution according to claim 11, wherein the pH is 10.0 or higher at 20°C.

## Patentansprüche

1. Verfahren zur Erzeugung einer flüssigen Zusammensetzung mit einem pH von 10,0 oder mehr, umfassend eine Verbindung 3, dargestellt durch die folgende Formel (3), und Wasser, durch Reaktion einer Verbindung 1, dargestellt durch die folgende Formel (1), und einer Verbindung 2, dargestellt durch die folgende Formel (2), in Wasser, das ein Verfahren zur Erzeugung einer flüssigen Zusammensetzung ist, umfassend Tetrahydro-4H-1,3,5-oxadiazin-4-on, worin
das molare Zuführverhältnis der Verbindung 2 zu der Verbindung 1, d.h., der Verbindung 2/der Verbindung 1, 0,3 oder höher und 1,7 oder weniger ist, und
die Reaktion zwischen der Verbindung 1 und der Verbindung 2 bei 15°C oder mehr und 50°C oder weniger und einem pH von 10,0 oder mehr in der Gegenwart von Alkyldiethanolamin durchgeführt wird

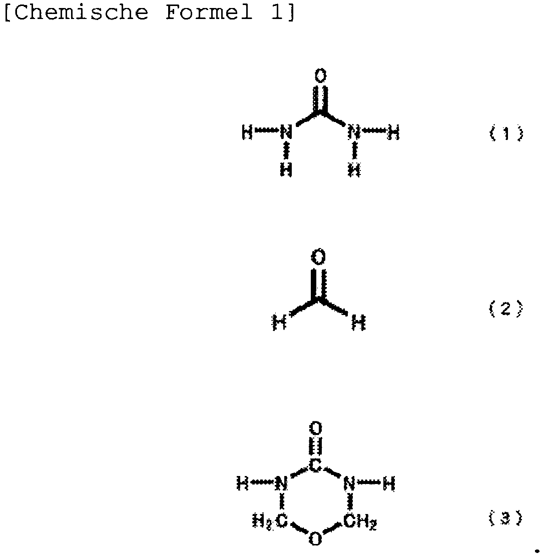

2. Verfahren zur Erzeugung einer flüssigen Zusammensetzung gemäß Anspruch 1, worin das molare Zuführverhältnis von Alkyldiethanolamin zu der Gesamtmenge der Verbindung 1 und der Verbindung 2, Alkyldiethanolamin/(Gesamtmenge der Verbindung 1 und der Verbindung 2) 0,005 oder mehr und 0,10 oder weniger ist.

3. Verfahren zur Erzeugung einer flüssigen Zusammensetzung gemäß Anspruch 1 oder 2, worin die Reaktion zwischen der Verbindung 1 und der Verbindung 2 durch Zugabe der Verbindung 2 zu einer Mischung durchgeführt wird, die die Verbindung 1, Alkyldiethanolamin und Wasser enthält.

4. Verfahren zur Erzeugung einer flüssigen Zusammensetzung gemäß einem der Ansprüche 1 bis 3, worin die Gesamtzuführmenge der Verbindung 1 und der Verbindung 2 3,0 Masse-% oder mehr und 25 Masse-% oder weniger in Bezug auf 100 Masse-% der Gesamtmenge der Verbindung 1, der Verbindung 2, Alkyldiethanolamin und Wasser ist.

5. Verfahren zur Erzeugung einer flüssigen Zusammensetzung gemäß einem der Ansprüche 1 bis 4, worin die Zuführmenge an Alkyldiethanolamin 0,1 Masse-% oder mehr und 5,0 Masse-% oder weniger in Bezug auf 100 Masse-% der Gesamtmenge der Verbindung 1, der Verbindung 2, Alkyldiethanolamin und Wasser ist.

6. Verfahren zur Erzeugung einer flüssigen Zusammensetzung gemäß einem der Ansprüche 1 bis 5, worin die Zuführmenge an Wasser 70 Masse-% oder mehr und 95 Masse-% oder weniger in Bezug auf 100 Masse-% der Gesamtmenge der Verbindung 1, der Verbindung 2, Alkyldiethanolamin und Wasser ist.

7. Verfahren zur Erzeugung einer flüssigen Zusammensetzung gemäß einem der Ansprüche 1 bis 6, worin die Reaktionszeit 1 Stunde oder länger und 4,5 Stunden oder weniger ist.

8. Verfahren zur Erzeugung einer flüssigen Zusammensetzung gemäß einem der Ansprüche 1 bis 7, worin der Gehalt der Verbindung 3 in der erhaltenen flüssigen Zusammensetzung 1,4 Masse-% oder mehr und 30 Masse-% oder weniger ist.

9. Verfahren zur Erzeugung einer flüssigen Zusammensetzung gemäß einem der Ansprüche 1 bis 8, worin Alkyldiethanolamin N-Methyldiethanolamin ist.

10. Verfahren zur Erzeugung einer flüssigen Zusammensetzung gemäß einem der Ansprüche 1 bis 9, worin die Reaktion zwischen der Verbindung 1 und der Verbindung 2 bei einem pH von 13,0 oder weniger durchgeführt wird.

11. Lagerungslösung einer flüssigen Zusammensetzung, erhalten durch das Verfahren zur Erzeugung einer flüssigen Zusammensetzung gemäß einem der Ansprüche 1 bis 10, worin die Lagerungslösung die Verbindung 3, Alkyldiethanolamin und Wasser enthält, worin der Gehalt von Wasser in der Lagerungslösung 70 Masse-% oder mehr ist und der pH 10,0 oder mehr und 12,5 oder weniger ist.

12. Lagerungslösung gemäß Anspruch 11, worin der pH 10,0 oder mehr bei 20°C ist.

## Revendications

1. Procédé de production, en faisant réagir un composé 1 représenté par la formule (1) suivante et un composé 2 représenté par la formule (2) suivante dans l'eau, d'une composition liquide avec un pH de 10,0 ou plus contenant un composé 3 représenté par la formule (3) suivante et de l'eau, qui est un procédé de production d'une composition liquide contenant de la tétrahydro-4H-1,3,5,-oxadiazin-4-one, dans lequel
le rapport d'alimentation molaire du composé 2 sur le composé 1, c'est-à-dire, le composé 2/le composé 1, est de 0,3 ou plus et de 1,7 ou moins, et
la réaction entre le composé 1 et le composé 2 est effectuée à 15 °C ou plus et 50 °C ou moins et à un pH de 10,0 ou plus en présence d'alkyldiéthanolamine.

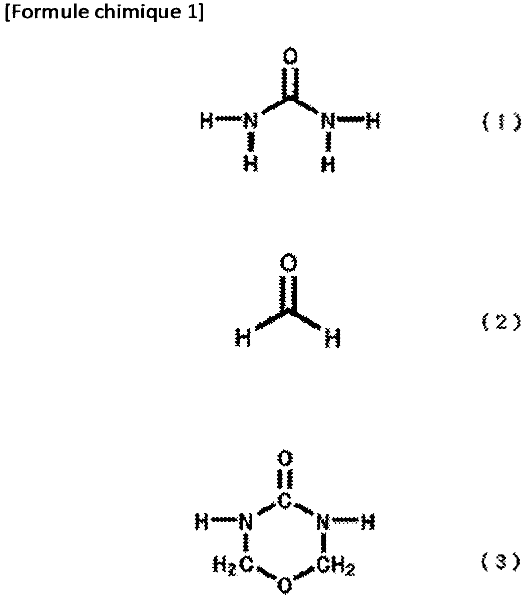

2. Procédé de production d'une composition liquide selon la revendication 1, dans lequel le rapport d'alimentation molaire de l'alkyldiéthanolamine sur la quantité totale du composé 1 et du composé 2, alkyldiéthanolamine/ (total du composé 1 et du composé 2), est de 0,005 ou plus et de 0,10 ou moins.

3. Procédé de production d'une composition liquide selon la revendication 1 ou 2, dans lequel la réaction entre le composé 1 et le composé 2 est effectuée en ajoutant le composé 2 à un mélange contenant le composé 1, l'alkyldiéthanolamine, et de l'eau.

4. Procédé de production d'une composition liquide selon l'une quelconque des revendications 1 à 3, dans lequel la quantité d'alimentation totale du composé 1 et du composé 2 est de 3,0 parties en masse ou plus et 25 parties en masse ou moins par rapport à 100 parties en masse de la quantité totale du composé 1, du composé 2, de l'alkyldiéthanolamine, et de l'eau.

5. Procédé de production d'une composition liquide selon l'une quelconque des revendications 1 à 4, dans lequel la quantité d'alimentation d'alkyldiéthanolamine est de 0,1 partie en masse ou plus et 5,0 parties en masse ou moins par rapport à 100 parties en masse de la quantité totale du composé 1, du composé 2, de l'alkyldiéthanolamine, et de l'eau.

6. Procédé de production d'une composition liquide selon l'une quelconque des revendications 1 à 5, dans lequel la quantité d'alimentation d'eau est de 70 parties en masse ou plus et 95 parties en masse ou moins par rapport à 100 parties en masse de la quantité totale du composé 1, du composé 2, de l'alkyldiéthanolamine, et de l'eau.

7. Procédé de production d'une composition liquide selon l'une quelconque des revendications 1 à 6, dans lequel le temps de réaction est de 1 heure ou plus long et 4,5 heures ou plus court.

8. Procédé de production d'une composition liquide selon l'une quelconque des revendications 1 à 7, dans lequel la teneur en composé 3 de la composition liquide obtenue est de 1,4 % en masse ou plus et 30 % en masse ou moins.

9. Procédé de production d'une composition liquide selon l'une quelconque des revendications 1 à 8, dans lequel l'alkyldiéthanolamine est la N-méthyl-diéthanolamine.

10. Procédé de production d'une composition liquide selon l'une quelconque des revendications 1 à 9, dans lequel la réaction entre le composé 1 et le composé 2 est effectuée à un pH de 13,0 ou moins.

11. Solution de stockage d'une composition liquide obtenue par le procédé de production d'une composition liquide selon l'une quelconque des revendications 1 à 10, la solution de stockage comprenant le composé 3, l'alkyldiéthanolamine et de l'eau, dans laquelle la teneur en eau de la solution de stockage est de 70 % en masse ou plus, et le pH est de 10,0 ou plus et 12,5 ou moins.

12. Solution de stockage selon la revendication 11, dans laquelle le pH est de 10,0 ou plus à 20 °C.
